# NEUE EUROPÄISCHE PATENTSCHRIFT

(11) **EP 3 431 117 B2**
(45) Veröffentlichungstag und Bekanntmachung des Hinweises auf die Entscheidung über den Einspruch: **22.02.2023**
(45) Hinweis auf die Patenterteilung: 15.07.2020
(21) Anmeldenummer: 18184208.9
(22) Anmeldetag: 18.07.2018
(51) Int. Cl.: A61M 1/14, A61J 1/10, A61J 1/14

(54) **ENTSORGUNGSBEHÄLTER FÜR VERBRAUCHTE DIALYSIERFLÜSSIGKEIT SOWIE EXTRAKORPORALE BLUTREINIGUNGSANLAGE MIT EINEM SOLCHEN ENTSORGUNGSBEHÄLTER**
DISPOSAL CONTAINER FOR SPENT DIALYSIS FLUID AND EXTRACORPOREAL BLOOD PURIFICATION INSTALLATION WITH SUCH A DISPOSAL CONTAINER
RÉCIPIENT DE COLLECTE POUR FLUIDES DE DIALYSE UTILISÉS AINSI QU'INSTALLATION DE TRAITEMENT EXTRACORPOREL DU SANG DOTÉE D'UN TEL RÉCIPIENT DE COLLECTE

(30) Priorität: 20.07.2017 DE 102017116394
(43) Veröffentlichungstag der Anmeldung: 23.01.2019
(73) Patentinhaber: B. Braun Avitum AG, 34212 Melsungen (DE)
(72) Erfinder: ROHDE, Alexander, 34212 Melsungen (DE)
(74) Vertreter: Winter, Brandl - Partnerschaft mbB

(56) Entgegenhaltungen:
- EP-A1- 0 715 860
- EP-A1- 2 818 152
- WO-A1-88/10124
- WO-A1-95/20985
- WO-A1-2013/050689
- JP-A- H09 323 597
- US-A- 3 515 275
- US-A- 6 039 060
- US-A1- 2004 267 183

## Beschreibung

Die vorliegende Erfindung betrifft einen Entsorgungsbehälter; vorzugsweise einen sterilen Entsorgungsbehälter, für verbrauchte Dialysierflüssigkeit gemäß dem Oberbegriff des Anspruchs 1. Außerdem ist die Erfindung auf eine extrakorporale Blutreinigungsanlage, insbesondere Dialyseanlage, gemäß dem Oberbegriff des Anspruchs 6 gerichtet. Gattungsgemäße Blutreinigungsanlagen weisen einen stationären Anlagenbereich, wie ein stationäres Abwassersystem, und einen selektiv daran anschließbaren instationären Anlagenbereich, wie zumindest eine Dialysemaschine der mobilen Bauart, auf.

### Hintergrund der Erfindung

Dialysemaschinen der mobilen Bauart, wie beispielsweise Maschinen für eine Akutdialyse, werden bei akutem Nierenversagen eines Patienten in allen Bereichen einer Klinik, insbesondere auf einer Intensivstation, eingesetzt. Hierbei ist eine schnelle/dynamische Einsatzfähigkeit der Dialysemaschine ein wichtiges Kriterium, weshalb vorgefertigte Dialysierlösungen an der Dialysemaschine angebracht sind. Die verbrauchte Dialysierflüssigkeit, das heißt die Dialysierflüssigkeit, die von der Dialysemaschine aufbereitet worden ist und einen Dialysator zur Blutreinigung eines Patienten durchlaufen hat, wird bei Dialysemaschinen der mobilen Bauart in einem (sterilen) Entsorgungsbehälter, der aus einem Beutel, wie einem Verwurfbeutel, besteht, gesammelt. Dieser ist gegenüber der Umgebung vorzugsweise steril abgedichtet, sodass auch die verbrauchte Dialysierflüssigkeit gegenüber der keimintensiven Umgebung, wie etwa der Intensivstation, abgeschottet ist.

Nach oder - je nach Volumen des Beutels - auch während der Behandlung, wird ein mit verbrauchter Dialysierflüssigkeit gefüllter Beutel entsorgt. Hierbei wird der Beutel zunächst in ein Ausgussbecken gegeben / geworfen, sodass der Beutelinhalt, das heißt die verbrauchte Dialysierflüssigkeit, in ein Abwassersystem der jeweiligen Klinik gelangt. Anschließend wird der leere Beutel, das heißt die von Dialysierflüssigkeit entleerte Beutelhülle, einem Sonderabfallsystem zugegeben. Dieses Sonderabfallsystem ist gesondert zu restlichem Abfall zu behandeln, weshalb dessen Entsorgung mit vergleichsweise hohen Kosten verbunden ist.

In der Praxis ist nun beobachtet worden, dass im Abfluss, nämlich dann, wenn die von der keimintensiven Klinikumgebung verunreinigte Beutelhülle mit der auslaufenden, verbrauchten Dialysierflüssigkeit in Kontakt gerät, die ins Abwassersystem abgeführte Dialysierflüssigkeit mit zusätzlichen Keinem belastet wird. So kann es passieren, dass etwa krankheitserregende Keime, die normalerweise über das vorstehend genannte Sonderabfallsystem zu entsorgen wären, in das Abwassersystem einer Klinik gelangen.

### Stand der Technik

Um jener Gefahr Einhalt zu gebieten, werden im Stand der Technik die Beutel, die in entsprechenden Ausgussbecken ausgeleert werden, in denen eine Vermischung mit etwa auf der Intensivstation eingefangenen Keimen möglich ist, mitsamt ihres Beutelinhalts, das bedeutet Beutelhülle plus verbrauchte Dialysierflüssigkeit, in ein Sonderabfallsystem gegeben.

Nachteilig an dieser Lösung ist, dass die Entsorgung des Sonderabfalls mit steigendem Gewicht einen erhöhten finanziellen Aufwand nach sich zieht. Außerdem birgt die Entsorgung von vollständig gefüllten Beuteln das Risiko einer Beutelleckage, wodurch sich die verbrauchte Dialysierflüssigkeit im Sonderabfall mit den Tücken einer Flüssigkeit ausbreitet.

Des Weiteren ist aus der WO 95/20985 A1 ein Entsorgungsbehälter bekannt, der mittels einer Leitung mit Konnektor in ein stationäres Abwassersystem entleert werden kann.

### Kurzbeschreibung der Erfindung

Der vorliegenden Erfindung liegt angesichts dieses Standes der Technik die Aufgabe zugrunde, die Nachteile aus dem Stand der Technik zu beheben oder zumindest zu mildern, und insbesondere den Beutelinhalt, das heißt die verbrauchte Dialysierflüssigkeit, in das Abwassersystem statt in das Sonderabfallsystem zu geben, jedoch unter Vermeidung der Gefahr einer Vermischung mit Keimen, die sich auf der Oberfläche des Beutels befinden.

Dies wird erfindungsgemäß mittels einer extrakorporale Blutreinigungsanlage mit den Merkmalen des Anspruchs 1 gelöst. Vorteilhafte Ausführungsformen sind Gegenstand der Unteransprüche.

Aus dieser erfindungsgemäßen Ausgestaltung eines Beutels sowie einer extrakorporalen Blutreinigungsanlage lassen sich beispielsweise folgende Vorteile ableiten:
- Ein erhöhter Hygienestandard aufgrund der unterbundenen Kontaminationsmöglichkeit der verbrauchten Dialysierflüssigkeit.
- Ein ergonomischer Vorteil für die Bediener der Blutreinigungsanlage, aufgrund des temporären Kurzschlusses zwischen Beutel und Abwassersystem.
- Ein ökonomischer Vorteil aufgrund der sinkenden Sonderabfallkosten.

Der Gegenstand der Erfindung ist demnach eine extrakorporale Blutreinigungsanlage mit einem stationären Anlagebereich, der zumindest eine Abwasserleitung eines Abwassersystems aufweist, und mit einem instationären Anlagebereich, der zumindest eine Blutreinigungsmaschine aufweist. Eine solche Blutreinigungsmaschine stellt eine Dialysierflüssigkeit parat, welche dazu vorbereitet ist, einen Dialysator zu durchlaufen, um somit beispielsweise das Blut eines Patienten zu reinigen. Nach dem Durchlaufen des Dialysators führt die Blutreinigungsmaschine die verbrauchte Dialysierflüssigkeit weiter in / an den Beutel, der vorzugsweise nach Art eines Einmalartikel-Verwurfsbeutels ausgestaltet ist.

Der Beutel ist demnach ein, vorzugsweise steriler, Entsorgungsbehälter für verbrauchte Dialysierflüssigkeit, aufweisend (oder sogar bestehend aus) einen flexiblen Beutel in oder an welchem ein Einlass-Anschluss zur Aufnahme der verbrauchten Dialysierflüssigkeit ausgebildet oder angeordnet ist. Der Einlass-Anschluss ist zum Anschluss des Beutels an eine Dialysemaschine der mobilen Bauart, das heißt eine "Akutmaschine" vorbereitet. Hieraus folgt, dass der etwa sterile Entsorgungsbehälter mit der Dialysemaschine bewegbar oder verfahrbar ist, sodass die Akutmaschine vielerorts ohne hohen Aufwand einsetzbar ist.

Erfindungsgemäß weist der Entsorgungsbehälter / der Beutel einen separat zum Einlass-Anschluss vorgesehenen Auslass-Anschluss oder Konnektor auf, der zum Anschluss des Beutels an eine Abwasserleitung, die in der Klinikumgebung vorhanden ist, in zur Konnektorumgebung dichter Weise, sodass kein Austausch zwischen dem Inneren des Konnektors und dem Äußeren des Konnektors möglich ist, vorbereitet ist. Als Teil des erfinderischen Gedankens ist der Beutel mittels eines Konnektors derart mit der Abwasserleitung (des Abwassersystems) verbindbar, dass die im Beutel befindliche, das bedeutet, vom Beutel erfasste / aufgenommene / verbrauchte Dialysierflüssigkeit über die Abwasserleitung aus der Blutreinigungsanlage und später aus der Klinik abtransportierbar ist. So lässt sich erreichen, dass der Beutelinhalt, das heißt die verbrauchte Dialysierflüssigkeit, unmittelbar und direkt in das Abwassersystem gelangt, ohne vorher potentielle Keime der Beuteloberfläche aufzusaugen. Das Risiko einer unerwünschten Kontamination des Abwasserstroms ist somit ausgeräumt.

Erfindungsgemäß weist der Konnektor der extrakorporalen Blutreinigungsanlage einen weiteren sogenannten Zusatzanschluss auf, über den der Konnektor mit einer Zusatz(wasser)leitung der extrakorporalen Blutreinigungsanlage koppelbar ist. Jene Zusatzleitung führt vorteilhafterweise Wasser in sich und zum Konnektor hin, sodass sie mittels einer Druckbeaufschlagung eine erhöhte Entleergeschwindigkeit der Dialysierflüssigkeit aus dem Beutel heraus in die Abwasserleitung hinein gewährleistet. So ist unter Ausnutzung der Prinzipien der Strömung nach Bernoulli und Venturi dem Ziel Folge geleistet, dass eine Dialysemaschine der mobilen Art unmittelbar nach ihrer Anwendung an einem ersten Ort an einem weiteren Ort wieder einsetzbar ist, da das Entleeren des Beutels aufgrund der Druckbeaufschlagung innerhalb kürzester Zeit von statten geht.

In anderen Worten lässt sich die Erfindung funktional derart beschreiben, dass der instationäre Anlagenbereich der Blutbehandlungsanlage mit dem stationären Anlagenbereich in eine solche Wirkverbindung gestellt wird, dass der Beutelinhalt (instationär) mit dem Abwassersystem (stationär) ohne Umwege und ohne Zwischenschaltung eines weiteren Elementes / Schrittes kommuniziert.

Strukturell gilt, dass eine Überbrückung zwischen dem Beutel (instationär) und der Abwasserleitung (stationär) mittels eines Konnektors, der von einem Bediener, vorzugsweise zu Beginn der Akutdialyse, angebracht / angelegt / in Betrieb genommen / gelegt wird, realisiert ist.

Neben dem Vorteil der Vermeidung einer Keimübertragung in das Abwassersystem hinein lässt die erfindungsgemäße Lösung auch das offene Ausleeren von Beuteln, wie Verwurfsbeuteln, überflüssig werden. Die hierbei entstehenden Spritzer und Verwirbelungen, die das Ausgussbecken wiederum mit der verbrauchten Dialysierflüssigkeit verschmutzen, treten erfindungsgemäß nicht mehr auf, wodurch der Hygienestandard in einer Klinik weiter erhöht wird.

Gemäß der Erfindung ist der Konnektor, der jene Überbrückung gewährleistet, integral / stoffeinstückig / einmaterialig mit dem Beutel ausgestaltet. So ist eine mögliche Übertragung der krankheitserregenden Keime, etwa von der Intensivstation, in das Beutelinnere und somit in die Abwasserleitung von vorne herein ausgeschlossen, wodurch höchste medizinische Hygienevorschriften ohne zusätzlichen Aufwand einzuhalten sind. Überdies weist der Konnektor an seinem beutelabgewandten Ende einen Anschluss, vorzugsweise nach Art eines Luer-Lock-Anschlusses oder eines Dreiwegehahns oder einer Waltherkupplung, auf, um diesen sicher und zeiteffizient mit der Abwasserleitung zu koppeln. Eine Waltherkupplung ist ein Anschlusssystem, bei dem ein festes Kupplungsteil gegenüber einem zur Längsachse des Anschlusses abschnittsweise verfahrbares Kupplungsteil bewegt wird, um ein Ein- bzw. Auskoppeln zu bewirken. Je nach Ausführungsform ist das feste Kupplungsteil anschlussseitig oder alternativ abwasserleitungsseitig und das verfahrbare Kupplungsteil jeweils komplementär zum Festen.

Grundsätzlich ist es hierfür auch möglich, einen Beutelanschluss in Form eines Luer-Lock-Anschluss vorzusehen. Hierbei wird beispielsweise der Innenkegel von dem Beutel ausgeformt und der Außenkegel von dem Konnektor. Anders herum ist es auch denkbar, den Außenkegel durch den Beutel auszuformen und den Innenkegel durch den Konnektor. Der Luer-Lock-Anschluss zeichnet sich durch ein hohes Maß an Zuverlässigkeit aus, wodurch hier eine Verunreinigung des Beutelinhalts ausgeschlossen wird.

Alternativ zum Luer-Lock-Anschluss ist neben einer Abflusskopplung auch eine beutelseitige Kopplung mittels einer Walterkupplung denkbar. Hierbei ist das feste Kupplungsteil wahlweise an dem Beutel oder dem Konnektor anbringbar. Das zum festen Kupplungsteil in Längsrichtung verfahrbare, bewegliche Kupplungsteil (dessen Verfahrweg ein Ein- und Auskoppeln ermöglicht), ist entsprechend komplementär zum festen Kupplungsteil ausgebildet.

Überdies ist es denkbar, die erfindungsgemäße extrakorporale Blutreinigungsanlage mit einem Klappmechanismus zu versehen, der dazu vorbereitet ist, eine Kompressionskraft auf den Beutel zu übertragen, um dessen Volumen zu verringern und somit mittels einer Druckbeaufschlagung eine erhöhte Entleergeschwindigkeit der Dialysierflüssigkeit aus dem Beutel heraus in die Abwasserleitung hinein zu gewährleisten. Hierbei wird somit mit einer Volumenveränderung anstelle einer Strömungsveränderung (wie in dem zuvor vorgestellten Ausführungsbeispiel vorgestellt) gearbeitet, um eine maximale Entleergeschwindigkeit zu erreichen.

Weiterhin zeichnet sich eine bevorzugte Ausführungsform dadurch aus, den Beutel von / mit einer Umverpackung / Hülle / Verpackung zumindest teilweise / abschnittsweise zu umgeben, sodass mittels jener Umverpackung eine zusätzliche Sterilbarriere zwischen der Umgebung und der äußeren Oberfläche des Beutels verwirklicht ist. Dies erhöht die Sicherheit der erfindungsgemäßen extrakorporalen Blutreinigungsanlage dadurch, dass es krankheitserregenden Keimen zusätzlich erschwert wird, den Beutel zu kontaminieren. Jene Umverpackung wird (ebenfalls) über den Sonderabfall einer Klinik entsorgt, wodurch dessen Kontamination keine hygienischen Probleme in der Entsorgung bewirkt.

Zusätzlich ist es vorteilhaft, wenn der Konnektor von einem verschieblichen Gehäuse zumindest teilweise / abschnittsweise umgeben ist und dieses Gehäuse erst unmittelbar vor dem Verbinden des Konnektors mit dem Beutel verschoben wird, sodass das Gehäuse eine zusätzliche Sterilbarriere zwischen der Umgebung und dem Konnektor darstellt. Auch dies erhöht die Sicherheit der erfindungsgemäßen extrakorporalen Blutreinigungsanlage dadurch, dass es krankheitserregenden Keimen zusätzlich erschwert wird, den Beutel zu kontaminieren. Jenes Gehäuse wird (ebenfalls) über den Sonderabfall einer Klinik entsorgt, wodurch dessen Kontamination keine hygienischen Probleme in der Entsorgung bewirkt.

Darüber hinaus ist in einer bevorzugten Ausführungsform die Abwasserleitung des Abwassersystems eine Ver- und/oder Entsorgungsleitung einer stationären Blutreinigungsanlage, vorzugsweise einer Blutreinigungsanlage für eine chronische Dialysebehandlung. Somit sind die Vorteile einer Dialysemaschine der mobilen Art zur Akutbehandlung, das heißt ein mobiler Einsatzort, mit den Vorteilen einer Dialysemaschine der stationären Art zur chronischen Dialysebehandlung, das heißt ein etabliertes Abwassersystem, miteinander vereint.

Die Erfindung wird nachstehend anhand bevorzugter Ausführungsbeispiele unter Bezugnahme auf die begleitenden Figuren näher erläutert. Die Figuren sind lediglich schematischer Natur und dienen ausschließlich dem Verständnis der Erfindung. Die gleichen Elemente sind mit denselben Bezugszeichen versehen.

Es zeigen:
- Fig. 1:: eine erfindungsgemäße extrakorporale Blutreinigungsanlage in schematischer Darstellung;
- Fig. 2a:: einen Beutel mit einem Konnektor in einer ersten Ausführungsform;
- Fig. 2b:: einen Beutel mit einem Konnektor in einer zweiten Ausführungsform;
- Fig. 3a:: einen Beutel mit einem Konnektor in einer weiteren Ausführungsform;
- Fig. 3b:: einen Konnektor in einer weiteren Ausführungsform für sich dargestellt;
- Fig. 4:: eine weitere Ausführungsform der erfindungsgemäßen Blutreinigungsanlage;
- Fig. 5:: einen schematisch dargestellten Beutel mit einem Klappmechanismus;
- Fig. 6: einen Beutel in einer weiteren Ausführungsform; und
- Fig. 7: einen Konnektor in einer weiteren Ausführungsform.

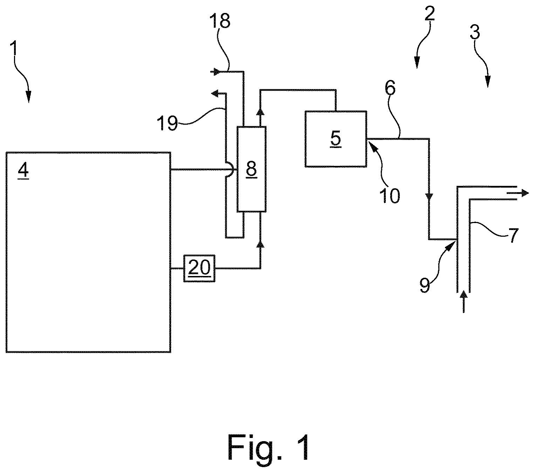

In Fig. 1 ist eine extrakorporale Blutreinigungsanlage 1 dargestellt. Diese weist einen instationären Anlagenbereich 2 auf, zu dem etwa eine Dialysemaschine 4, vorzugsweise der mobilen Art, gehört. Daneben weist die extrakorporale Blutreinigungsanlage 1 einen stationären Anlagenbereich 3 auf, zu dem etwa die stationären Leitungen eines Abwassersystems, wie eine Abwasserleitung 7 gehören. Zu dem instationären Anlagenbereich 2 können auch - anders als in Fig. 1 dargestellt - mehrere Dialysemaschinen zählen, die dann über mehrere Anschlüsse mit der Abwasserleitung 7 verbunden sind.

Als wesentlicher Bestandteil der Erfindung ist ein Beutel 5, wie ein Einmalartikel-Verwurfsbeutel, ein sogenannter Disposable, einem Dialysator 8 nachgeschaltet. In dem Beutel 5 wird somit eine von der Dialyse verbrauchte Dialysierflüssigkeit gesammelt. Als Brücke zwischen dem instationären Anlagenbereich 2 und dem stationären Anlagebereich 3 ist ein Konnektor 6 angeordnet, der es ermöglicht, die verbrauchte Dialysierflüssigkeit aus dem Beutel 5 in die Abwasserleitung 7 zu transferieren / übergeben / weiterzuleiten. Somit ist der Beutel 5 mittels des Konnektors 6 und der Abwasserleitung 7 rechtzeitig und unmittelbar entleert und muss nicht an ein externes Ausgussbecken weitergegeben werden, sondern ist, nach der Beendigung der Dialyse, direkt in den Sonderabfall zu geben. Einer möglichen Kontamination in beide Richtungen - also sowohl von Keimen in der Umgebung auf die verbrauchte Dialysierflüssigkeit und somit in das Abwassersystem, als auch von der verbrauchten Dialysierflüssigkeit in die Umgebung - ist somit vorgebeugt.

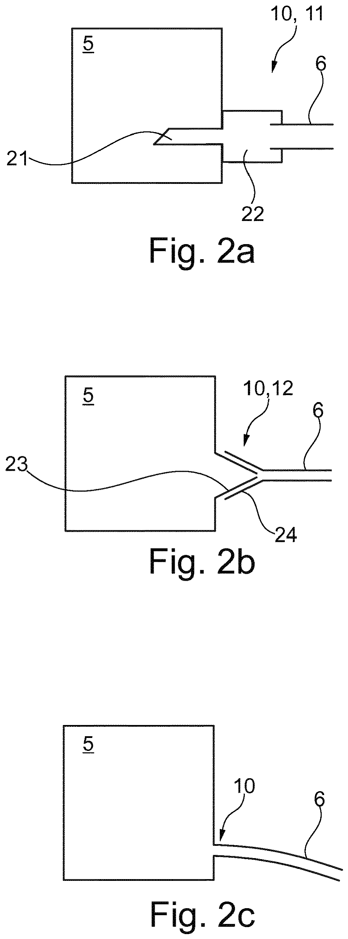

Der Konnektor 6 ist über einen Beutelanschluss 10 mit dem Beutel 5 verbunden. Einzelne Ausführungsformen des Beutelanschlusses 10 werden im Zusammenhang mit den Figuren 2a bis 2c näher vorgestellt. Am anderen Ende des Konnektors 6 ist ein beutelabgewandter Anschluss 9 angeordnet. Dieser garantiert eine sichere, günstige und zeiteffiziente Verbindung des Konnektors 6 mit der Abwasserleitung 7. Als Beispiele für den beutelabgewandten Anschluss 9 seien ein Luer-Lock-Anschluss 12 oder ein Dreiwegehahn oder eine Waltherkupplung genannt.

Die grundsätzliche Funktionsweise einer extrakorporalen Blutreinigungsanlage 1 der gattungsgemäßen Art, nämlich dass Blut von einem Patienten über eine erste Leitung 18 dem Dialysator 8, vorzugsweise im Gegenstromprinzip betrieben, zugeführt wird, wo es gereinigt wird und dem Patienten wieder über eine zweite Leitung 19 zurückgeführt wird, ist bekannt, weshalb diesbezüglich auf den Stand der Technik verwiesen sei. Erwähnenswert an dieser Stelle ist lediglich, dass die extrakorporale Blutreinigungsanlage 1 eine Pumpe 20 aufweist, die die unverbrauchte Dialysierflüssigkeit zum Dialysator 8 und von dort die verbrauchte Dialysierflüssigkeit weiter in den Beutel 5 fördert.

Unter Bezugnahme auf Fig. 2a sei eine erste Ausführungsform des Beutelanschlusses 10 vorgestellt.

Hierbei ist der Beutelanschluss 10 als ein Luer-Lock-Anschluss 12 realisiert (alternativ wäre, wie bereits erwähnt, auch eine Walterkupplung denkbar). So bildet der Beutel 5 etwa einen Innenkegel 23 aus, auf den ein von dem Konnektor 6 ausgestalteter Außenkegel 24 aufgesetzt wird, um eine sichere Verbindung zwischen dem Beutel 5 und dem Konnektor 6 zu realisieren. Der Außenkegel 24 ist entweder integral mit dem Konnektor 6 ausgestaltet oder als ein zusätzliches Teil auf diesen aufgesetzt.

Eine zweite Möglichkeit des Beutelanschlusses 10 ist in Fig. 2b dargestellt. Hierbei weist der Beutel 5 stoffeinstückig den Beutelanschluss 10 auf. Auf diese Weise sind der Konnektor 6 und der Beutel 5 als ein Bauteil ausgestaltet, was logistische Vorteile bewirkt, da der Beutelanschluss 10 nicht mehr nachträglich anzubringen ist.

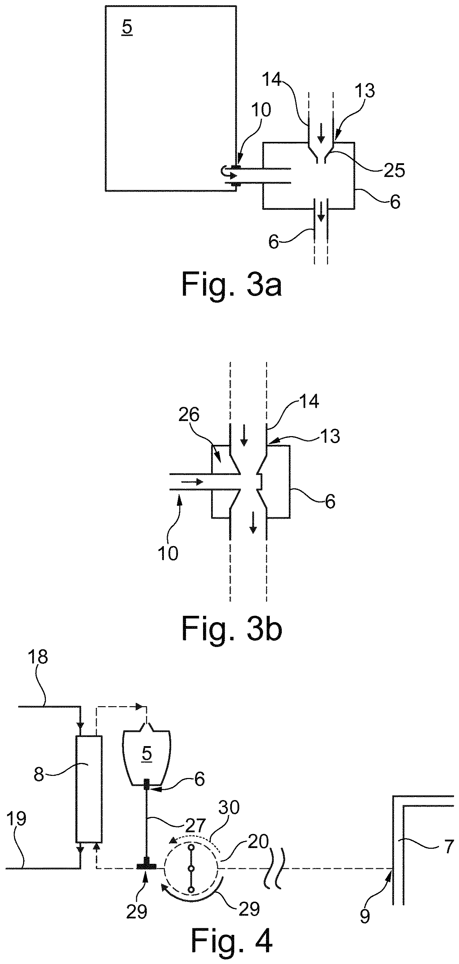

Eine weitere Ausführungsform des Zusammenspiels aus Beutel 5 und Konnektor 6 ist in Fig. 3a dargestellt. Der Konnektor 6 ist hierbei über einen Zusatzanschluss 13 mit einer Zusatzleitung 14 gekoppelt und realisiert somit eine Wasserstrahlpumpe. Die Zusatzleitung 14 führt vornehmlich Wasser entlang der mittels des oberen Pfeils dargestellten Strömungsrichtung mit sich. Aufgrund der Verjüngung 25 der Zusatzleitung 14 wird eine Geschwindigkeitserhöhung des in der Zusatzleitung 14 geführten Wassers erreicht, was eine Druckabnahme nach sich zieht (Bernoulli). Jene Druckabnahme führt zu einem "Saugen" der verbrauchten Dialysierflüssigkeit aus dem Beutel 5 (siehe Pfeil im Beutel 5) heraus, was zu einem schnellen und effizienten Entleeren des Beutels 5 führt. Mittels der Ausführungsform mit dem Zusatzanschluss 13 und der Zusatzleitung 14 ist demnach eine schnelle Entleerung des Beutels 5 erreicht. Der Konnektor 6 weist weiterhin eine Leitung auf (siehe unterer Pfeil), die die Mischung aus verbrauchter Dialysierflüssigkeit (aus dem Beutel 5) und Wasser (aus der Zusatzleitung 14) in Richtung der Abwasserleitung 7 transportiert (vgl. Fig. 1). Jene Leitung ist entweder als integraler Bestandteil des Konnektors 6 oder als ein zusätzliches Bauteil ausgestaltbar.

In Fig. 3b ist ein ähnliches Funktionsprinzip mittels einer Zusatzleitung 14 und einem Zusatzanschluss 13 dargestellt. Abweichend von Fig. 3a wird hierbei eine Venturi-Düse 26 eingesetzt. Das Wirkprinzip der Venturi-Düse 26 ist ebenfalls auf Bernoulli zurückzuführen und sei deshalb an dieser Stelle - da bereits im Zusammenhang mit Fig. 3a geschehen - nicht tiefergehend erläutert.

Fig. 4 zeigt eine weitere Ausführungsform der Erfindung. Hierbei ist die Pumpe 20 als Peristaltikpumpe angedeutet. Der Konnektor 6 ist unter Zwischenschaltung einer Überbrückungsleitung 27 und einer Dialysierflüssigkeitsleitung 28 mit der Abwasserleitung 7 gekoppelt. Hierfür ist ein Dreiwegehahn 29 angeordnet. In einem ersten Zustand, in dem die Dialyse stattfindet, ist der Dreiwegehahn 29 in einer solchen Position, dass kein Fluid durch die Überbrückungsleitung 27 gefördert wird. Die Pumpe 20 dreht sich entlang der ersten Förderrichtung 29. Bei Betrachtung des schematisch dargestellten Aufbaus in Fig. 4 ist ersichtlich, dass ein Einlass-Anschluss, vorzugsweise im oberen Bereich des Beutels 5 angeordnet ist.

Sobald die Dialyse abgeschlossen ist, wird der Dreiwegehahn 29 gedreht, sodass der Pfad zwischen der Überbrückungsleitung 27 und der Leitung, in der die Pumpe 20 angeordnet ist, freigegeben ist. In diesem Zustand wird die Pumpe nun rückwärts laufen gelassen, das heißt entlang der zweiten Förderrichtung 30. Somit ist unter Zuhilfenahme der Pumpleistung der Pumpe 20 eine Förderung der verbrauchten Dialyiserflüssigkeit aus dem Beutel 5 heraus zur Abwasserleitung 7 hin ermöglicht.

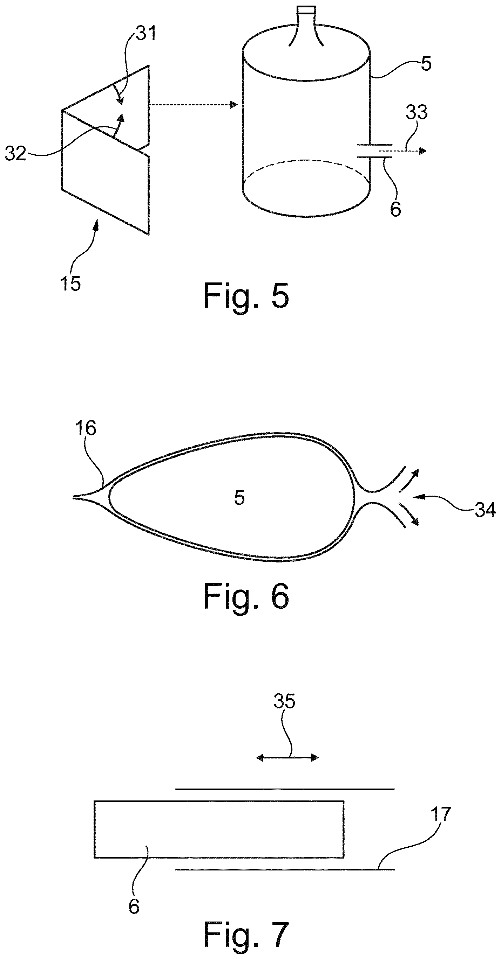

In Fig. 5 ist ein weiterer Mechanismus zum Entleeren des Beutels 5 offenbart. Im Gegensatz zu den zuvor vorgestellten Mechanismen, die einen Unterdruck erzeugen, wird hier ein Klappmechanismus 15 eingesetzt, um das Beutelvolumen durch mechanische Kraft von außen zu verringern. In dem lediglich schematisch angedeuteten Klappmechanismus 15 aus Fig. 5 sind zwei Verschwenkpfeile 31, 32 erkennbar, die den Beutel 5 nach Art einer Aluminium-Dose, die im Recycle-Vorgang komprimiert wird, zerdrücken, um so den Volumenstrom entlang des Pfeils 33 zu erzwingen. Dies gewährleistet ein schnelles Entleeren des Beutels 5.

In Fig. 6 ist ein Beutel 5 schematisch dargestellt. Dieser ist von einer Umverpackung 16 umgeben, die eine zusätzliche Sterilbarriere realisiert. Die Umverpackung 16 weist eine offene Einschnürung 34 auf, an der ein Bediener mittels einer entgegengesetzten Bewegung die Umverpackung 16 lösen kann. Dies beugt einer Kontamination der Außenfläche des Beutels 5 vor.

Fig. 7 stellt ein Ausführungsbeispiel dar, in dem ein Gehäuse 17 um den Konnektor 6 herum angeordnet ist. Das Gehäuse 17 kann den Konnektor 6 ganz oder auch nur teilweise umgeben. Entlang einer Schiebebewegung 35 ist das Gehäuse 17, etwa entgegen der Vorspannung einer Feder, relativ zum Konnektor 6 verschiebbar. Somit ist es möglich, dass der Konnektor 6, etwa in der Ausführungsform, in der er als Einsteckdorn 11 ausgestaltet ist (vgl. Fig. 2a) erst unmittelbar vor dem Einstechen / Einstecken mit der von Keimen angelagerten Umgebungsluft (etwa auf der Intensivstation) in Kontakt kommt, wodurch mittels des Gehäuses 17 eine zusätzliche Sterilbarriere realisiert ist, welche die von der erfindungsgemäßen Lösung erreichten Hygienestandards weiter erhöht.

**Bezugszeichenliste**

| | | | |
|---|---|---|---|
| 1 | Extrakorporale | 18 | Erste Leitung |
| | Blutreinigungsanlage | 19 | Zweite Leitung |
| 2 | Instationärer Anlagebereich | 20 | Pumpe |
| 3 | Stationärer Anlagebereich | 21 | Spitze |
| 4 | Blutreinigungsmaschine | 22 | Sitz |
| 5 | Beutel | 23 | Innenkegel |
| 6 | Konnektor | 24 | Außenkegel |
| 7 | Abwasserleitung | 25 | Verjüngung |
| 8 | Dialysator | 26 | Venturi-Düse |
| 9 | Beutelabgewandter | 27 | Überbrückungsleitung |
| | Anschluss | 28 | Dialysierflüssigkeitsleitung |
| 10 | Beutelanschluss | 29 | Erste Förderrichtung |
| 11 | Einsteckdorn | 30 | Zweite Förderrichtung |
| 12 | Luer-Lock-Anschluss | 31 | Erster Verschwenkpfeil |
| 13 | Zusatzanschluss | 32 | Zweiter Verschwenkpfeil |
| 14 | Zusatzleitung | 33 | Volumenstrom |
| 15 | Klappmechanismus | 34 | Offene Einschnürung |
| 16 | Umverpackung | 35 | Schiebebewegung |
| 17 | Gehäuse | | |

## Patentansprüche

1. Extrakorporale Blutreinigungsanlage (1) mit
einem eine Abwasserleitung (7) aufweisenden stationären Anlagebereich (3) und mit
einem instationären Anlagebereich (2), der zumindest eine Blutreinigungsmaschine (4) aufweist, welche dazu vorbereitet ist, eine Dialysierflüssigkeit nach deren Durchlaufen eines Dialysators (8) an einen Entsorgungsbehälter, vorzugsweise nach Art eines Einmalartikel-Verwurfsbeutels, weiterzugeben,
wobei der Entsorgungsbehälter für verbrauchte Dialysierflüssigkeit, einen flexiblen Beutel (5) aufweist in oder an welchem ein Einlass-Anschluss ausgebildet oder angeordnet ist, der zum Anschluss des Beutels (5) an eine Dialysemaschine der mobilen Bauart vorbereitet ist, derart, dass der, vorzugsweise sterile, Entsorgungsbehälter mit der Dialysemaschine bewegbar oder verfahrbar ist, und
einen separat zum Einlass-Anschluss vorgesehenen Auslass-Anschluss oder Konnektor (6) aufweist, der zum Anschluss des Beutels (5) an eine Abwasserleitung (7) in zur Konnektorumgebung dichter Weise vorbereitet ist, wobei
der Konnektor (6) integral mit dem Beutel (5) ausgestaltet ist und an seinem beutelabgewandten Ende einen Anschluss (9), vorzugsweise nach Art eines Luer-Lock-Anschlusses (12) oder eines Dreiwegehahns oder einer Waltherkupplung, aufweist, um mit der Abwasserleitung (7) koppelbar zu sein, **dadurch gekennzeichnet, dass**
der Konnektor (6) einen Zusatzanschluss (13) aufweist, über den der Konnektor (6) mit einer Zusatzleitung (14) der Blutreinigungsanlage (1) koppelbar ist, die dazu vorbereitet ist, mittels einer Druckbeaufschlagung eine erhöhte Entleergeschwindigkeit der Dialysierflüssigkeit aus dem Beutel (5) in die Abwasserleitung (7) zu gewährleisten.

2. Extrakorporale Blutreinigungsanlage (1) nach einem der vorstehenden Ansprüche, **dadurch gekennzeichnet, dass** ein Klappmechanismus (15) dazu vorbereitet ist, eine Kompressionskraft auf den Beutel (5) zu übertragen, um dessen Volumen zu verringern und somit mittels einer Druckbeaufschlagung eine erhöhte Entleergeschwindigkeit der Dialysierflüssigkeit aus dem Beutel (5) in die Abwasserleitung (7) zu gewährleisten.

3. Extrakorporale Blutreinigungsanlage (1) nach einem der vorstehenden Ansprüche, **dadurch gekennzeichnet, dass** der Beutel (5) von einer Umverpackung (16) zumindest teilweise umgeben ist, sodass die Umverpackung (16) eine Sterilbarriere zwischen der Umgebung und der äußeren Oberfläche des Beutels (5) darstellt.

4. Extrakorporale Blutreinigungsanlage (1) nach einem der vorstehenden Ansprüche, **dadurch gekennzeichnet, dass** der Konnektor (6) von einem verschieblichen Gehäuse (17) zumindest teilweise umgeben ist und das Gehäuse (17) unmittelbar vor dem Verbinden verschoben wird, sodass das Gehäuse (17) eine Sterilbarriere zwischen der Umgebung und dem Konnektor (6) darstellt.

5. Extrakorporale Blutreinigungsanlage (1) nach einem der vorstehenden Ansprüche, **dadurch gekennzeichnet, dass** die Abwasserleitung (7) einer Ver- und/oder Entsorgungsleitung einer stationären Blutreinigungsanlage, vorzugsweise einer Blutreinigungsanlage für eine chronische Dialysebehandlung, entspricht.

## Claims

1. An extracorporeal blood purification system (1) comprising
a stationary system area (3) including a sewer line (7) and comprising
a non-stationary system area (2) including at least one blood purification machine (4) which is prepared to pass a dialysate, after the latter has flown through a dialyser (8), on to a disposal container, preferably in the form of a single-use reject bag,
wherein the disposal container for used dialysate comprises a flexible bag (5) in or at which an inlet connection is formed or arranged which is prepared for connecting the bag (5) to a dialysis machine of the mobile type such that the, preferably sterile, disposal container is movable or displaceable along with the dialysis machine, and comprises an outlet connection or connector (6) which is provided separately from the inlet connection and is prepared for connecting the bag (5) to a sewer line (7) in a way sealed against the connector environment, wherein
the connector (6) is formed integrally with the bag (5) and at its end facing away from the bag includes a connection (9), preferably in the form of a Luer lock (12) or a three-way cock or a Walther coupling, so as to be adapted to be coupled to the sewer line (7),
**characterized in that**
the connector (6) includes an auxiliary connection (13) via which the connector (6) can be coupled to an auxiliary line (14) of the blood purification system (1) which is prepared to ensure an increased emptying rate of the dialysate from the bag (5) into the sewer line (7) by means of pressurization.

2. The extracorporeal blood purification system (1) according to any one of the preceding claims, **characterized in that** a folding mechanism (15) is prepared to transmit a compressing force to the bag (5) so as to reduce the volume thereof and thus to ensure an increased emptying rate of the dialysate from the bag (5) into the sewer line (7).

3. The extracorporeal blood purification system (1) according to any one of the preceding claims, **characterized in that** the bag (5) is at least partly surrounded by an outer packaging (16) so that the outer packaging (16) constitutes a sterile barrier between the environment and the outer surface of the bag (5).

4. The extracorporeal blood purification system (1) according to any one of the preceding claims, **characterized in that** the connector (6) is at least partly surrounded by a movable housing (17) and the housing (17) is displaced immediately before connecting so that the housing (17) constitutes a sterile barrier between the environment and the connector (6).

5. The extracorporeal blood purification system (1) according to any one of the preceding claims, **characterized in that** the sewer line (7) corresponds to a supply and/or disposal line of a stationary blood purification system, preferably a blood purification system for chronical dialysis treatment.

## Revendications

1. Dispositif de filtration du sang extracorporelle (1) muni d'une zone d'appui stationnaire (3) présentant une conduite d'évacuation (7) et d'une zone d'appui non stationnaire (2), qui présente au moins une machine de filtration du sang (4), laquelle est prévue pour transférer un liquide de dialyse après son passage à travers un dialyseur (8) vers un récipient d'élimination, de préférence de type poche de rebut jetable,
dans laquelle le récipient d'élimination pour liquide de dialyse usagé présente une poche souple (5) dans ou sur laquelle est formé ou agencé un orifice d'entrée, qui est prévu pour raccorder la poche (5) à une machine de dialyse de type mobile, de sorte que de préférence le récipient d'élimination stérile peut être déplacé ou transporté avec la machine de dialyse, et
un orifice de sortie disposé de manière distincte par rapport à l'orifice d'entrée ou un raccord (6), qui est prévu pour le raccordement de la poche (5) à une conduite d'évacuation (7) de manière étanche à proximité du raccord,
dans lequel le raccord (6) est conçu d'un seul tenant avec la poche (5) et présente à son extrémité opposée de la poche un orifice (9), de préférence de type raccord Luer-Lock (12) ou robinet à trois voies ou raccordement Walther pour pouvoir être couplé à la conduite d'évacuation (7),
**caractérisé en ce que**
le raccord (6) présente un orifice supplémentaire (13), par le biais duquel le raccord (6) peut être couplé avec une conduite supplémentaire (14) du dispositif de filtration du sang (1) qui est prévu pour garantir au moyen d'une pressurisation une accélération de la vitesse de vidage du liquide de dialyse de la poche (5) dans la conduite d'évacuation (7).

2. Dispositif de filtration du sang extracorporelle (1) selon l'une quelconque des revendications précédentes, **caractérisé en ce qu'**un mécanisme de rabattement (15) est prévu pour transférer une force de compression sur la poche (5) afin de diminuer son volume et, par conséquent, pour garantir au moyen d'une pressurisation une accélération de la vitesse de vidage du liquide de dialyse de la poche (5) dans la conduite d'évacuation (7).

3. Dispositif de filtration du sang extracorporelle (1) selon l'une quelconque des revendications précédentes, **caractérisé en ce que** la poche (5) est enveloppée au moins partiellement d'un emballage (16), de sorte que l'emballage (16) représente une barrière stérile entre l'environnement et la surface externe de la poche (5).

4. Dispositif de filtration du sang extracorporelle (1) selon l'une quelconque des revendications précédentes, **caractérisé en ce que** le raccord (6) est enveloppé au moins partiellement d'un logement coulissant (17) et **en ce que** le logement (17) est déplacé immédiatement avant le raccordement, de sorte que le logement (17) représente une barrière stérile entre l'environnement et le raccord (6).

5. Dispositif de filtration du sang extracorporelle (1) selon l'une quelconque des revendications précédentes, **caractérisé en ce que** la conduite d'évacuation (7) correspond à une conduite d'alimentation et/ou une conduite d'élimination d'un dispositif de filtration du sang stationnaire, de préférence d'un dispositif de filtration du sang pour un traitement par dialyse chronique.
